# EUROPÄISCHE PATENTANMELDUNG

(11) **EP 0 981 262 A2**
(43) Veröffentlichungstag der Anmeldung: **23.02.2000**
(21) Anmeldenummer: 98121614.6
(22) Anmeldetag: 12.11.1998
(51) Int. Cl.: H04R 25/00

(54) **Implantat**

(30) Priorität: 20.08.1998 DE 19837863
(71) Anmelder: IMPLEX GmbH Spezialhörgeräte, D-85737 Ismaning (DE)
(72) Erfinder: Fiedler, Dirk A. Dr., 80805 München (DE)
(74) Vertreter: Schwan, Gerhard, Dipl.-Ing.

(57) **Zusammenfassung**

Um die Lebensdauer von Implantaten, die selbst elektrische Energie verbrauchen oder mit einer elektrische Energie verbrauchenden Anordnung gekoppelt sind, zu verlängern, wird vorgeschlagen, bei derartigen Implantaten eine elektrische Energiequelle vorzusehen, die mindestens eine von der Anode und der Kathode der elektrischen Energiequelle unabhängige Potentialsonde aufweist. Auf diese Weise lassen sich Implantate realisieren, bei welchen unter Anwendung einer geeigneten Steuerung eine Verkürzung der Lebensdauer der in der elektrischen Energiequelle eingesetzten Elektroden durch Vermeidung von die Elektroden schädigenden Prozessen verhindert und somit die Lebensdauer des Implantats selbst verlängert werden kann.

## Beschreibung

Die vorliegende Erfindung betrifft ein Implantat mit einer elektrische Energie verbrauchenden Anordnung und einer eine Anode und eine Kathode aufweisenden elektrischen Energiequelle, welche mit der elektrische Energie verbrauchenden Anordnung gekoppelt ist. Vorliegend wird unter einem derartigen Implantat ein Implantat verstanden, welches entweder selbst elektrische Energie verbraucht und/oder welches mit einer separaten, jedoch gleichfalls implantierten, elektrische Energie verbrauchenden Anordnung gekoppelt ist.

Bei derzeit verfügbaren Implantaten, die zum Betrieb eine elektrische Energiequelle benötigen wie beispielsweise Herzschrittmachern, Hörgeräten, Stimulationsgeräten und dergleichen werden als elektrische Energiequelle entweder Primärzellen oder Sekundärzellen eingesetzt. Eine die Funktion des Implantats gefährdende Abnahme der Leistungsfähigkeit der elektrischen Energiequelle läßt sich dadurch vermeiden, daß die Zelle bereits vor Ablauf der zu erwartenden Betriebsdauer ausgetauscht bzw. nachgeladen wird. Da jedoch jeder Austausch der elektrischen Energiequelle eine Operation des Implantatträgers erforderlich macht, ist das Erreichen einer langen Betriebs- und Lebensdauer der elektrischen Energiequelle von vorrangiger Bedeutung.

Um einerseits die Leistungsfähigkeit der in dem Implantat vorgesehenen elektrischen Energiequelle, sei es als Primär- oder als Sekundärzelle, vorhersagen zu können sowie um andererseits die einzelnen Elektroden schädigenden Prozesse, wie sie insbesondere bei Ladevorgängen einer als Sekundärzelle ausgeführten elektrischen Energiequelle auftreten können, zu verhindern, sollten die Elektroden hinsichtlich bestimmter Kenngrößen wie Strom und Spannung überwacht werden.

Bei einem mit einer konventionellen elektrischen Energiequelle ausgestatteten Implantat lassen sich die Elektroden der elektrischen Energiequelle nicht unabhängig voneinander bzw. individuell beobachten und kontrollieren. Vielmehr sind die außerhalb der Energiequelle meßbaren Kenngrößen Strom und Spannung immer auf die gesamte Kombination der in der elektrischen Energiequelle vorgesehenen Elektroden bezogen. Bei einer Messung dieser Kenngrößen verläßt man sich im allgemeinen darauf, daß die Elektroden beim Entladen, im Ruhezustand und gegebenenfalls beim Laden vorhersagbare Eigenschaften haben. Eine solche Messung kann jedoch durch gleichzeitig ablaufende, die Elektroden unterschiedlich polarisierende, Vorgänge verfälscht werden und erlaubt dann nur noch bedingt und in genauer Kenntnis dieser Vorgänge unter den momentan geltenden Randbedingungen Rückschlüsse auf den momentanen Zustand der elektrischen Energiequelle.

Beispielsweise werden beim Laden einer elektrochemischen Sekundärzelle die Gleichgewichtspotentiale der beiden aktiven Elektroden aufgrund der existenten Innenwiderstände zu negativeren (negative Elektrode) und positiveren (positive Elektrode) Potentialen hin verschoben. Die Innenwiderstände setzen sich dabei aus ohmschen und nicht-ohmschen Teilen zusammen. Ohmsche Anteile betreffen in der Regel Kontakt- und Elektrolytwiderstände. Nicht-ohmsche Anteile sind durch Elektrodenbeschaffenheit und die an den Elektroden ablaufenden elektrochemischen Vorgänge gegeben.

Insgesamt ergibt sich ein sehr komplexes Netzwerk von resistiven, kapazitiven und induktiven Komponenten, das insbesondere im Lastfall, d.h. wenn die elektrische Energiequelle das Implantat mit elektrischer Energie versorgt, nicht mehr aufgelöst werden kann. Aus einer einfachen Strom-/Spannungsmessung läßt sich daher keine Aussage mehr dahingehend ableiten, welche der beteiligten Elektroden sich nach Wunsch verhalten und welche nicht.

Nur durch umfangreiche Erfahrung mit einem gegebenen System unter klar definierten Randbedingungen (z.B. 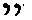 Entladen mit ^{C}/₂ bis Entladeschlußspannung 1,5 V"; 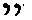 Laden mit ^{C}/₁₀ für 14 h") ist für den Fachmann abschätzbar, ob sich aufgrund der gemessenen Strom- und Spannungswerte die betrachtete elektrische Energiequelle 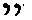 gut" oder 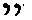 schlecht" verhält. Ist weiterhin für eine gegebene elektrische Energiequelle das Entladeverhalten bei einer bestimmten Strombelastung bei einem bestimmten Abschaltkriterium bekannt, läßt sich aus diesem Verhalten keinesfalls exakt vorhersagen, wie sich die elektrische Energiequelle bei z.B. 1/10 oder 1/100 dieser Strombelastung verhalten wird. Bestenfalls kann der Fachmann eine Abschätzung geben.

Es ist daher eine Aufgabe der vorliegenden Erfindung, ein Implantat zu schaffen, welches eine genauere und zuverlässigere Messung und/oder Überwachung von Elektrodenkenngrößen erlaubt.

Diese Aufgabe wird erfindungsgemäß durch ein Implantat der eingangs genannten Art gelöst, bei welchem die elektrische Energiequelle mindestens eine von der Anode und der Kathode unabhängige Potentialsonde aufweist. Auf diese Weise erhält man ein von der Anode und der Kathode der elektrischen Energiequelle unabhängiges Bezugspotential, mittels dessen sich durch gezielte Überwachung und/oder Regelung einzelner Elektrodenpotentiale relativ zu dem Bezugspotential ungewollte Nebenreaktionen oder ungewollt übermäßig ablaufende Nebenreaktionen an den betrachteten Elektroden erkennen und verhindern lassen.

Somit kann bei Kenntnis der jeweiligen Elektrodeneigenschaften grundsätzlich verhindert werden, daß Elektroden irreversibel geschädigt werden, was zu einem frühzeitigen Ausfall der elektrischen Energiequelle führen könnte. Bei dem Implantat nach der Erfindung ist es also nicht mehr notwendig, umfassendes und auf langjähriger Erfahrung beruhendes Fachwissen mit langwierigen Testreihen zu kombinieren. Vielmehr sind bei dem erfindungsgemäßen Implantat nach wenigen und relativ zeitunkritischen Messungen definitive und allgemeingültige Aussagen hinsichtlich der betreffenden Elektroden möglich. Elektrodenschädigende Prozesse lassen sich somit einfach vermeiden, ohne daß eine auf vielen Annahmen beruhende Analyse der jeweiligen gesamten Strom/Spannungskurven durchgeführt werden müßte. Aufgrund der daraus resultierenden längeren Lebensdauer der in der elektrischen Energiequelle benutzten Elektroden wird ein vorzeitiger Zugriff auf das Implantat, der eine Operation des Implantatträgers erfordern würde, vermieden.

Weitere vorteilhafte Ausgestaltungen der Erfindung ergeben sich aus den Unteransprüchen.

Insbesondere kann die elektrische Energiequelle eine elektrochemische Energiequelle oder ein Superkondensator sein.

Handelt es sich bei der elektrischen Energiequelle um eine elektrochemische Energiequelle, so kann diese als galvanisches Element, insbesondere als Primärelement oder Sekundärelement, oder als Brennstoffzelle ausgeführt sein.

Des weiteren kann die elektrische Energiequelle des Implantats mit einem elektrisch leitenden Gehäuse versehen sein, welches einen Abgriff aufweist, der als Potentialsonde dient. Diese Ausführungsform stellt aus fertigungstechnischen Gründen die am einfachsten zu bewerkstelligende Lösung dar, da hierbei ein Abgriff von außen an dem Gehäuse der elektrischen Energiequelle z.B. mittels Löten angebracht werden kann, ohne daß Durchführungen in das Gehäuse erforderlich wären.

Hierbei kann das Gehäuse mehrere gegeneinander elektrisch isolierte Abschnitte aufweisen, wobei mindestens zwei der Gehäuseabschnitte über einen Abgriff verfügen, die als Potentialsonden dienen. Beispielsweise kann das Gehäuse der in dem Implantat vorgesehenen elektrischen Energiequelle einen ersten, die Anode umgebenden Gehäuseabschnitt sowie einen zweiten, die Kathode umgebenden Gehäuseabschnitt aufweisen, der gegenüber dem ersten Gehäuseabschnitt elektrisch isoliert ist, wobei der erste und der zweite Gehäuseabschnitt jeweils einen Abgriff aufweisen, die als Potentialsonden dienen. Bei dieser Ausgestaltung des erfindungsgemäßen Implantats liefern die Abgriffe zusätzlich zu ihrer Funktion des Bereitstellens von Bezugspotentialen für Messungen der Anode und der Kathode ferner Informationen über den Zustand des Inneren der elektrischen Energiequelle des Implantats an verschiedenen Bereichen innerhalb des Gehäuses der elektrischen Energiequelle.

Ist in weiterer Ausgestaltung der Erfindung bei dem vorgenannten Gehäuse zwischen dem ersten Gehäuseabschnitt und dem zweiten Gehäuseabschnitt ein gegenüber dem ersten und dem zweiten Gehäuseabschnitt elektrisch isolierter dritter Gehäuseabschnitt vorgesehen, wobei der erste, zweite und dritte Gehäuseabschnitt jeweils einen Abgriff aufweisen, die als Potentialsonden dienen, so lassen sich mittels der jeweiligen Abgriffe die Potentiale der jeweiligen Gehäuseabschnitte messen und somit Informationen über den Zustand der einzelnen Bereiche der elektrischen Energiequelle des Implantats gewinnen.

Ist ferner das Gehäuse gegenüber dem Gehäuseinneren elektrisch isoliert, so ergibt sich ein nach außen hin elektrisch neutrales Gehäuse.

Gemäß einer weiteren bevorzugten Ausgestaltung der Erfindung kann außer der Anode und der Kathode mindestens eine weitere als Potentialsonde dienende Elektrode vorgesehen sein, mit der sich das Potential eines zwischen der Anode und der Kathode befindlichen Elektrolyten abgreifen läßt.

Vorzugsweise ist das Implantat ferner mit einer Telemetrieeinrichtung versehen, um Daten zwischen dem Implantat und einer externen Meß- und/oder Steuervorrichtung zu übertragen. Derartige Telemetrieeinrichtungen, bei welchen Datensignale mittels magnetischer Induktion oder per Infrarotübertragung übermittelt werden, sind an sich aus dem Stand der Technik bekannt und bereits bei zahlreichen Implantaten im Einsatz.

Bevorzugte Ausführungsformen der vorliegenden Erfindung werden nachstehend unter Bezugnahme aufdie beigefügten Zeichnungen beschrieben, wobei
- FIG. 1: eine schematische Schnittansicht eines Implantats zeigt; und
- FIG. 2: bis FIG. 6 Schnittansichten von bei dem Implantat aus FIG. 1 eingesetzten elektrischen Energiequellen zeigen.

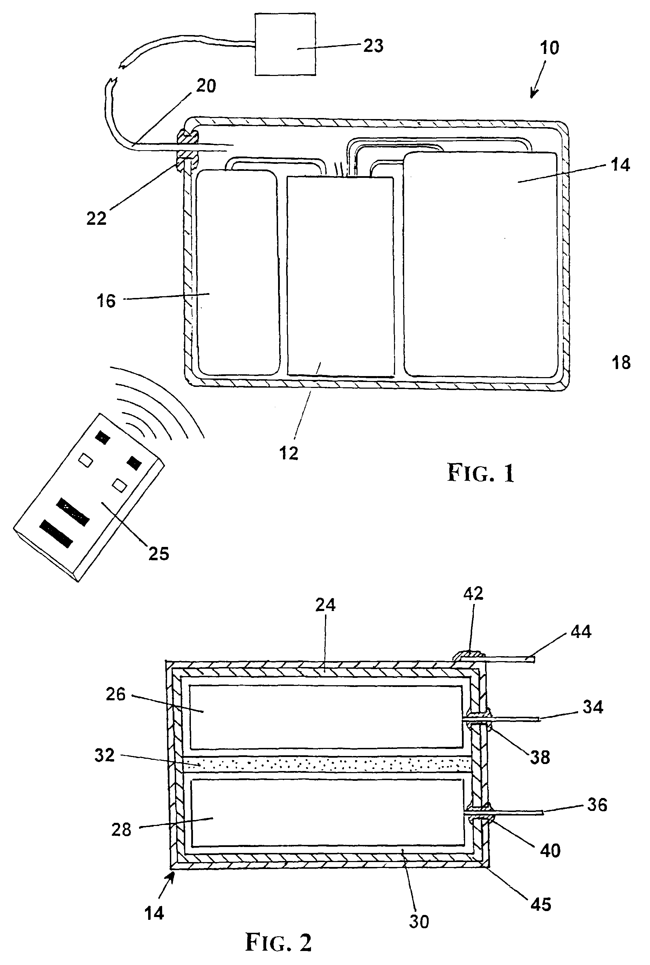

Das in FIG. 1 gezeigte Implantat 10 weist als Hauptbestandteile eine Steuereinheit 12, eine elektrische Energiequelle 14 sowie eine Telemetrieeinrichtung 16 auf, die alle in einem gemeinsamen Implantatgehäuse 18 untergebracht und über Leitungen miteinander verbunden sind. An die Steuereinheit 12 ist ferner eine Leitung 20 angeschlossen, die über eine Durchführung 22 aus dem Implantatgehäuse 18 herausgeführt ist und zu einem nur schematisch als Block 23 dargestellten aktiven Element führt, welches die jeweils gewünschte Implantatfunktion ausführt. Bei dem aktiven Element kann es sich beispielsweise um den Aktor eines vollimplantierten Hörgeräts, um Stimulationselektroden, um Medikamentenabgabevorrichtungen oder dergleichen handeln. Über die Telemetrieeinrichtung 16 kann das Implantat Abfragesignale oder Steuersignale von einer externen Meß- und/oder Steueranordnung 25 empfangen und Datensignale an diese übermitteln. Handelt es sich bei der elektrischen Energiequelle 14 um eine nachladbare Energiequelle, so dient die Telemetrieeinrichtung 16 ferner dem Empfang von von der Steueranordnung 25 abgesendeten Stromsignalen zum Nachladen der elektrischen Energiequelle 16.

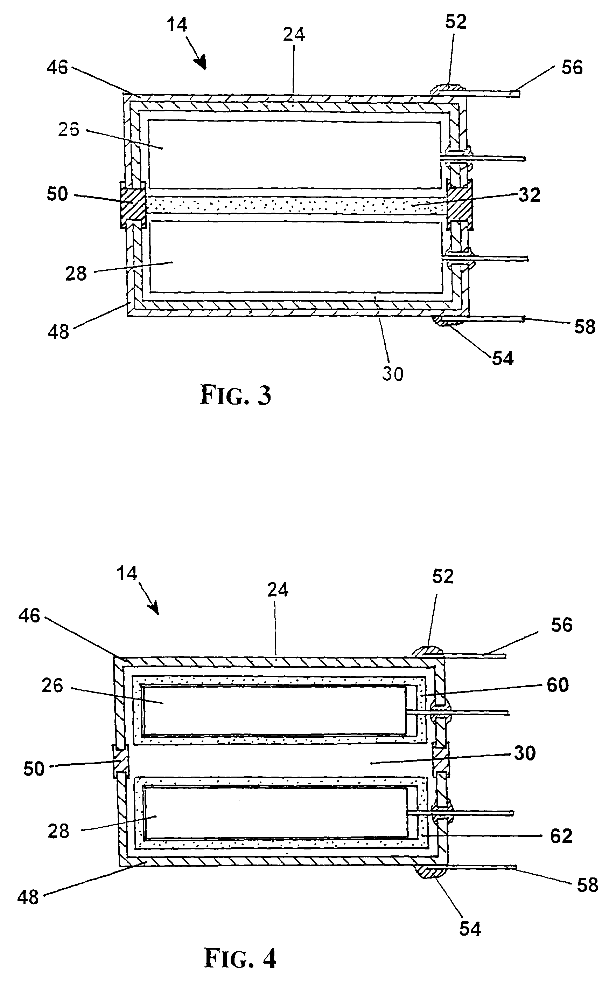

FIG. 2 zeigt im Detail eine elektrische Energiequelle 14 gemäß einer ersten Ausführungsform des vorliegend beschriebenen Implantats. Hierbei sind in einem elektrisch leitfähigen, vorzugsweise hermetisch dichten Gehäuse 24, welches beispielsweise aus Metall gefertigt ist, zwei Elektroden 26 und 28 angeordnet. Obschon es für die Funktion des vorliegend beschriebenen Implantats gleichgültig ist, welche der beiden Elektroden 26 und 28 die Anode ist und welche die Kathode, wird in der vorliegenden Beschreibung aus Gründen der Einfachheit die Elektrode 26 als Anode und die Elektrode 28 als Kathode bezeichnet. Der Innenraum des Gehäuses 24 ist mit einem Elektrolyten 30 gefüllt, wobei die Anode 26 und die Kathode 28 durch ein Diaphragma 32 voneinander getrennt sind. Das Diaphragma 32, bei dem es sich beispielsweise um einen mikroporösen Kunststoffscheider handeln kann, ist ein elektrischer Isolator, erlaubt jedoch eine Ionenwanderung zwischen den beiden Elektroden 26 und 28. Die Mode 26 und die Kathode 28 sind gegenüber dem elektrisch leitfähigen Gehäuse 24 elektrisch isoliert, beispielsweise mittels einer auf die Innenseite der Gehäusewand aufgebrachten isolierenden Schicht 45. Die Anode 26 und die Kathode 28 sind ferner über Leitungen 34 und 36, die über Durchführungen 38 bzw. 40 aus dem Gehäuse 24 herausgeführt sind, mit der Steuereinheit 12 verbunden, wie dies in FIG. 1 angedeutet ist. An dem Gehäuse 24 ist ein Abgriff 42 vorgesehen, an dem ein Referenzpotential abgegriffen werden kann. Handelt es sich um ein metallisches Gehäuse, so kann der Abgriff 42 als eine Leitung 44 ausgeführt sein, die mit der Außenseite des Gehäuses 24 leitend verbunden, beispielsweise an diese angelötet ist, wie dies in FIG. 2 veranschaulicht ist. Hierbei ist das Gehäuse potentialfrei, bzw. kann an der Leitung 44 ein Null-Potential als Referenz zu dem Anodenpotential und dem Kathodenpotential abgegriffen werden.

Ist die elektrische Energiequelle 14 eine elektrochemische Primärzelle, so können jegliche gebräuchlichen Elektroden-/Elektrolytsysteme verwendet werden, wie z.B. Zn/AgO, Zn/MnO₂, auf Lithium basierende Zellen, organische Systeme und solche mit flüssigen nieder- oder hochviskosen Elektrolyten sowie Festelektrolytsysteme. Ist hingegen die elektrische Energiequelle 14 als elektrochemische Sekundärzelle ausgeführt, so können Metall/ Luft-Akkumulatoren wie z.B. Zink/Luft-Systeme, Zn/MnO₂-Systeme, Nickel-Cadmium-Zellen, Nickel-Metallhydrid-Systeme, oder aber Lithium-Zellen eingesetzt werden. Unter Lithium-Zellen seien vorliegend Zellen zu verstehen, bei welchen eine Festkörperkathode aus Einlagerungsverbindungen zusammen mit einer Anode aus metallischen Lithium in Kombination mit einem flüssigen, organischen Elektrolyten oder einem Elektrolyten aus festem Polymer eingesetzt werden, sowie Lithium-Ionen-Zellen mit flüssigem oder festem Polymer-Elektrolyten, Lithium-Legierungszellen und dergleichen.

Wird als Elektrolyt ein Polymerelektrolyt verwendet, der zusätzlich zu seiner Funktion als Ionenleiter gleichzeitig auch eine Separatorfünktion übernimmt, so kann auf das in FIG. 2 gezeigte Diaphragma 32 verzichtet werden. Derartige Polymerelektrolyte können in Form von echten Polymerelektrolyten vorliegen, oder in Form eines mikroporösen Polymers, in dessen Poren eine Elektrolytlösung eingebracht ist.

Bei der in FIG. 2 gezeigten 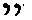 Drei-Elektroden-Anordnung" können durch eine Vergleichsmessung der Potentiale an den Leitungen 34 und 44 bzw. 36 und 44 die stromführenden Elektroden 26 und 28 unabhängig voneinander beobachtet werden.

Wenn das Meßproblem dies erfordert, können auch mehrere Potentialsonden eingesetzt werden. Bei einer derart abgewandelten Ausführungsform einer in dem Implantat 10 eingesetzten elektrochemischen Energiequelle 14, wie sie in FIG. 3 dargestellt ist, ist das elektrisch leitende Gehäuse 24 in zwei Gehäuseabschnitte 46 und 48 unterteilt, welche die in einem Elektrolyten 30 angeordneten, durch ein Diaphragma 32 voneinander getrennten Elektroden 26 und 28 umgeben. Zwischen den Gehäuseabschnitten 46 und 48 ist ein Isolator 50 vorgesehen, und an jedem der Gehäuseabschnitte 46 und 48 ist ein Abgriff 52 bzw. 54 bereitgestellt, an welchem über eine Leitung 56 bzw. 58 das Potential des jeweiligen Gehäuseabschnitts abgegriffen werden kann, um als Bezugspotential bei der Bewertung der elektrischen Energiequelle zu sorgen.

Bei der in FIG. 4 dargestellten abgewandelten Ausführungsform einer implantatseitigen elektrochemischen Energiequelle sind anstelle des in Fig. 3 gezeigten Diaphragmas, welches das Gehäuseinnere in zwei die Elektroden aufnehmende Bereiche unterteilt, die Anode 26 und die Kathode 28 jeweils von einem Diaphragma 60 bzw. 62 umgeben, welches zwar eine Ionenwanderung von und zu der Anode bzw. der Kathode zuläßt, das jedoch einen elektrischen Isolator darstellt, d.h. eine Elektronenwanderung verhindert. Insbesondere wenn das Gehäuse der elektrischen Energiequelle 14, das wie das in FIG. 3 gezeigte Gehäuse durch einen umlaufenden Isolator 50 in zwei Gehäuseabschnitte 46 und 48 unterteilt sein kann, aus Metall oder einem anderen leitfähigen Material gefertigt ist, wird bei dieser Ausgestaltung effektiv ein Kurzschluß zwischen der Anode bzw. der Kathode und dem Gehäuse verhindert.

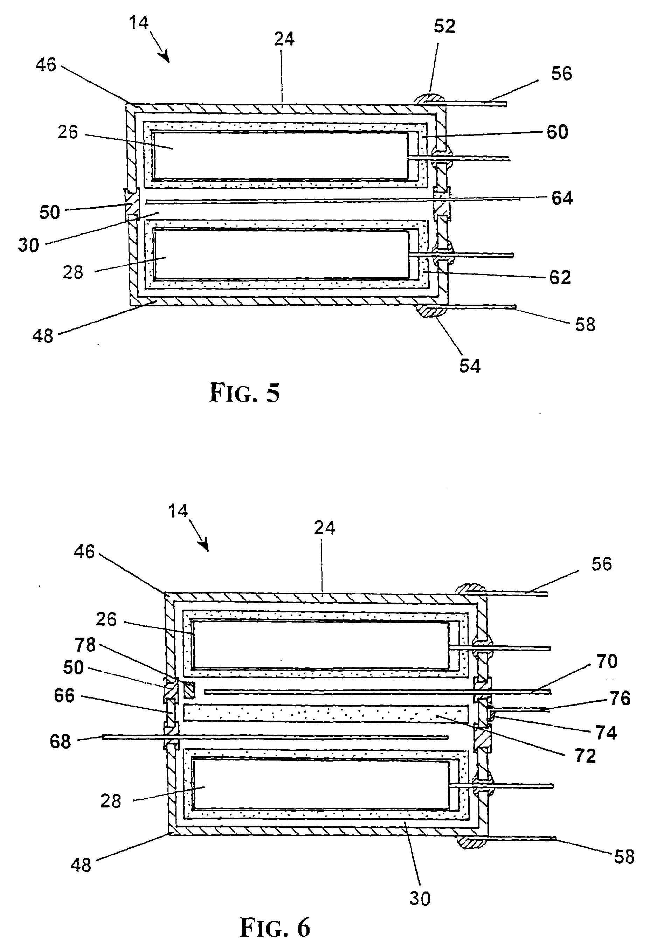

Die Ausführungsform einer implantatseitigen elektrochemischen Energiequelle gemäß FIG. 5 ist mit drei von der Anode 26 und der Kathode 28 unabhängigen Potentialsonden ausgestattet. Zwei dieser Potentialsonden werden hier wiederum von an Abgriffen 52 und 54 angebrachten Leitungen 56 und 58 gebildet, mittels deren die Potentiale der Gehäuseabschnitte 46 und 48 ermittelt werden können, während ein dritte Potentialsonde 64 im Elektrolyten 30 zwischen den beiden Elektroden 26 und 28 angeordnet ist. Handelt es sich bei dem Gehäuse 24 um ein elektrisch leitfähiges Gehäuse, so muß für eine Isolation zwischen der dritten Potentialsonde 64 und dem Gehäuse gesorgt sein, was gemäß FIG. 5 dadurch bewerkstelligt sein kann, daß die dritte Potentialsonde 64 durch den Isolator 50 geführt ist. Es versteht sich jedoch, daß die Potentialsonde auch an einer beliebigen anderen Stelle durch das Gehäuse 24 hindurch geführt sein kann, sofern für eine geeignete Isolation gesorgt ist. Eine Durchführung durch ein elektrisch leitfähiges Gehäuse läßt sich beispielsweise dadurch erzeugen, daß das betreffende zu isolierende Bauteil, insbesondere die Zuleitung der Potentialsonde oder mindestens eine der zu den Elektroden 26, 28 führenden Leitungen, in einem in dem Gehäuse vorgesehenen Durchbruch in Al₂O₃ vergossen wird. Anstelle für jede der aus dem Gehäuse 24 herausgeführten Leitungen jeweils eine eigene Durchführung vorzusehen, können ferner zwei oder mehr dieser Leitungen in einer gemeinsamen Durchführung zusammengefaßt werden.

FIG. 6 zeigt eine noch weiter abgewandelte Ausführungsform einer elektrochemischen Energiequelle, wie sie in dem vorliegend beschriebenen Implantat 10 verwendet wird. Das Gehäuse 24 ist hierbei in drei Gehäuseabschnitte unterteilt, einen ersten die Anode 26 umgebenden Gehäuseabschnitt 46, einen zweiten die Kathode 28 umgebenden Gehäuseabschnitt 48 sowie einen dritten zwischen dem ersten und zweiten Gehäuseabschnitt angeordneten Gehäuseabschnitt 66. Zwischen den Gehäuseabschnitten ist jeweils ein Isolator 50 angeordnet. Im Raum zwischen der Anode 26 und der Kathode 28 sind hierbei zwei Potentialsonden 68 und 70 angeordnet, die mittels eines Diaphragmas 72 elektrisch gegeneinander isoliert sind. Handelt es sich bei dem Gehäuse 24 um ein elektrisch leitfähiges Gehäuse, so können die Potentialsonden 68 und 70 analog zu FIG. 5 durch die Isolatoren 50 geführt sein, um für eine Isolation zwischen den Potentialsonden und dem Gehäuse zu sorgen.

Bei der in FIG. 6 dargestellten Ausführungsform einer implantatseitigen elektrochemischen Energiequelle 14 ist ferner der dritte Gehäuseabschnitt 66 mit einem Abgriff 74 versehen, an welchem über eine Leitung 76 das Potential des dritten Gehäuseabschnitts abgegriffen werden kann.

Es versteht sich, daß die vorliegend beschriebenen Ausführungsformen in beliebiger Weise miteinander kombiniert werden können, sofern für eine geeignete elektrische Isolation zwischen den einzelnen elektrisch leitfähigen Bauteilen, insbesondere den Elektroden, den Potentialsonden und gegebenenfalls dem Gehäuse, gesorgt ist. So ist in FIG. 6 ein Isolator 78 angedeutet, um die Potentialsonde 70 gegen das elektrisch leitfähige Gehäuse 24 abzuschirmen. Ein derartiger Isolator wird bei der in FIG. 6 gezeigten Ausführungsform zwar eigentlich nicht benötigt, da dessen Funktion von dem zwischen dem ersten und dem dritten Gehäuseabschnitt angeordneten isolierenden Verbindungsstück 50 bereits erfüllt wird, ist aber dann erforderlich, wenn die Potentialsonde an einer Stelle eingesetzt wird, wo kein derartiger Isolator bereits in die Gehäusewand integriert ist.

Da bei dem vorstehend beschriebenen Implantat bereits zum Zeitpunkt der Produktion Meßsonden zur Verfügung stehen, lassen sich lange vor dem geplanten Einsatz Kontrollmessungen durchführen, die, abhängig von der verwendeten Technologie, zu dann noch durchführbaren Nachbesserungen Anlaß geben können. Kontrollmessungen können dabei einfache Messungen von Potentialdifferenzen zwischen der Bezugselektrode und einer aktiven Elektrode sein. Aber auch komplexere Meßverfahren, z.B. (cyclo)voltammetrische Untersuchungen mit Gleichstrom- oder aber kombinierten Gleich-/Wechselstromanregungssignalen sowie impedanzspektroskopische Messungen und allgemein alle dem Fachmann geläufigen elektroanalytischen Methoden, die der jeweiligen Untersuchung oder Prüfung dienlich sind, können mit dem geschilderten Aufbau der in dem Implantat vorgesehenen elektrischen Energiequelle in Kombination mit kommerziell erhältlichen Meßapparaturen durchgeführt werden. Der Zeitpunkt solcher Messungen wird bereits während des Produktionsablaufs anzusetzen sein. Bis zum Einsatz des Implantats können derartige Messungen zur Kontrolle der Stabilität und Einsatzfähigkeit durchgeführt werden, Besondere Aufmerksamkeit kommt solchermaßen erfaßbaren Elektrodenpotentialen jedoch während des Einsatzes zu. Beispielsweise während eines Entladevorgangs wird es nun erstmals möglich, anhand des Verhaltens derjenigen Elektrode, die zuerst in eine unerwünschte oder schädliche Potentialregion gerät, den Entladevorgang zu unterbrechen. Folgeprozesse können dann sinnvoll und den Gegebenheiten entsprechend eingeleitet werden. Während des Ladens kann ebenso gezielt unterbrochen werden, wenn bereits eine Elektrode in einen unerwünschten oder sogar schädlichen Potentialbereich gerät. Es sei nochmals betont, daß gerade dieser Fall bei konventionellen Zwei-Elektroden-Anordnungen nicht zwingend erfaßt wird, und somit ohne Wissen und vor allem Kontrollmöglichkeit des Benutzers die elektrische Energiequelle des Implantats irreversibel geschädigt werden kann, was sich grundsätzlich negativ auf die Gesamtlebensdauer des Implantats auswirkt.

Das vorliegend beschriebene Konzept, bei einem Implantat eine elektrische Energiequelle einzusetzen, bei welcher zwecks Ermittlung und Überwachung des Zustandes der Energiequelle eine oder mehrere Potentialmessungen zusätzlich zu der Erfassung der einzelnen Elektrodenpotentiale möglich sind, läßt sich wie oben erwähnt ferner auch mit Superkondensatoren insbesondere mit Doppelschichtkondensatoren, Redoxkondensatoren oder Pseudokondensatoren, sowie mit Brennstoffzellen realisieren.

## Patentansprüche

1. Implantat mit einer elektrische Energie verbrauchenden Anordnung (23) und einer eine Anode (26) und eine Kathode (28) aufweisenden elektrischen Energiequelle (14), welche mit der elektrische Energie verbrauchenden Anordnung gekoppelt ist, dadurch gekennzeichnet, daß die elektrische Energiequelle (14) mindestens eine von der Anode (26) und der Kathode (28) unabhängige Potentialsonde (44, 56, 58, 68, 70, 76) aufweist.

2. Implantat nach Anspruch 1, dadurch gekennzeichnet, daß die elektrische Energiequelle (14) eine elektrochemische Energiequelle ist.

3. Implantat nach Anspruch 2, dadurch gekennzeichnet, daß die elektrochemische Energiequelle (14) ein galvanisches Element ist.

4. Implantat nach Anspruch 2, dadurch gekennzeichnet, daß die elektrochemische Energiequelle (14) eine Brennstoffzelle ist.

5. Implantat nach Anspruch 1, dadurch gekennzeichnet, daß die elektrische Energiequelle (14) ein Superkondensator ist.

6. Implantat nach einem der vorhergehenden Ansprüche, dadurch gekennzeichnet, daß die elektrische Energiequelle (14) mit einem elektrisch leitenden Gehäuse (24) versehen ist, welches einen Abgriff (42, 52, 54, 74) aufweist, der als Potentialsonde dient.

7. Implantat nach Anspruch 6, dadurch gekennzeichnet, daß das Gehäuse (24) mehrere gegeneinander elektrisch isolierte Abschnitte (46, 48, 66) aufweist und mindestens zwei der Gehäuseabschnitte einen Abgriff (42, 52, 54, 74) aufweisen, der als Potentialsonden dient.

8. Implantat nach Anspruch 7, dadurch gekennzeichnet, daß das Gehäuse (24) einen ersten, die Anode (26) umgebenden Gehäuseabschnitt (46) sowie einen zweiten, die Kathode (28) umgebenden Gehäuseabschnitt (48) aufweist, der gegenüber dem ersten Gehäuseabschnitt elektrisch isoliert ist, wobei der erste und der zweite Gehäuseabschnitt jeweils einen Abgriff (52, 54) aufweisen, die als Potentialsonden dienen.

9. Implantat nach Anspruch 8, dadurch gekennzeichnet, daß zwischen dem ersten Gehäuseabschnitt (46) und dem zweiten Gehäuseabschnitt (48) ein gegenüber dem ersten und dem zweiten Gehäuseabschnitt elektrisch isolierter dritter Gehäuseabschnitt (66) vorgesehen ist, wobei der erste, zweite und dritte Gehäuseabschnitt jeweils einen Abgriff (52, 54, 74) aufweisen, die als Potentialsonden dienen.

10. Implantat nach einem der Ansprüche 6 bis 9, dadurch gekennzeichnet, daß das Gehäuse (24) gegenüber dem Gehäuseinneren elektrisch isoliert ist.

11. Implantat nach einem der Ansprüche 6 bis 10, wobei die elektrische Energiequelle (14) ein galvanisches Element ist, dadurch gekennzeichnet, daß außer der Anode (26) und der Kathode (28) mindestens eine weitere als Potentialsonde dienende Elektrode (64; 68, 70) vorgesehen ist, mit der sich das Potential eines zwischen der Anode und der Kathode befindlichen Elektrolyten (30) abgreifen läßt.

12. Implantat nach einem der Ansprüche 1 bis 11, ferner versehen mit einer Telemetrieeinrichtung (16) zum Übertragen von Daten zwischen dem Implantat (10) und einer externen Meß- und/oder Steuervorrichtung (25).
